(19) Europäisches Patentamt
European Patent Office
Office européen des brevets

(11) Veröffentlichungsnummer: **0 216 166**
**A1**

(12) **EUROPÄISCHE PATENTANMELDUNG**

(21) Anmeldenummer: 86111730.7

(22) Anmeldetag: 25.08.86

(51) Int. Cl.4: C07D 213/68 , C07D 213/89 , C07D 401/04 , A01N 43/40 , A01N 43/48 , A01N 43/50 , A01N 43/647 , //C07D231/16

(30) Priorität: 06.09.85 DE 3531773

(43) Veröffentlichungstag der Anmeldung: 01.04.87 Patentblatt 87/14

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Knops, Hans-Joachim, Dr.
Köpenicker Strasse 35
D-4019 Monheim(DE)
Erfinder: Babczinski, Peter, Dr.
In der Lohrenbeck 11
D-5600 Wuppertal 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)
Erfinder: Lürssen, Klaus, Dr.
August-Kierspel-Strasse 151
D-5060 Bergisch Gladbach 2(DE)

(54) **3,5-Disubstituierte 4-Pyridone.**

(57) Die Erfindung betrifft neue 3,5-disubstituierte 4-Pyridone der Formel (I)

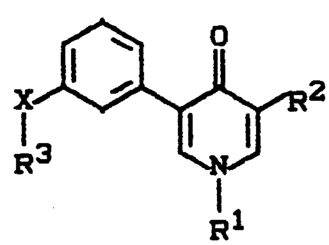

(I)

in welcher

$R^1$ für Alkyl, Alkenyl, Alkinyl, Alkoxy, Alkoxyalkyl oder Dialkylamino steht,

$R^2$ für gegebenenfalls substituiertes Triazolyl, Imidazolyl, Pyrazolyl oder Phenyl steht,

$R^3$ für Halogenalkyl oder Halogenphenyl steht, und

X für Sauerstoff oder Schwefel steht,

ein Verfahren zu ihrer Herstellung und ihre Verwen-

EP 0 216 166 A1

dung als Herbizide und Pflanzenwachstumsregulatoren.

# 3,5-Disubstituierte 4-Pyridone

Die vorliegende Erfindung betrifft neue 3,5-disubstituierte 4-Pyridone, ein Verfahren zu ihrer Herstellung und ihre Verwendung als Herbizide, insbesondere als selektive Herbizide und als Pflanzenwachstumsregulatoren.

Es ist bereits bekannt geworden, daß zahlreiche 3,5 disubstituierte 4-Pyridone, wie beispielsweise 1-Methyl-3-phenyl-5-(3-trifluormethylphenyl)-4-pyridon, als Herbizid, wie insbesondere auch zur selektiven Unkrautbekämpfung in Baumwolle eingesetzt werden können (vergleiche DE-OS 25 37 753). Der selektive Einsatz in verschiedenen anderen Kulturpflanzen-beständer ist jedoch nur bedingt möglich, da es dort zu Schäden kommen kann.

Über eine Wirksamkeit als Wachstumsregulatoren ist in diesem Zusammenhang nichts bekannt.

Es wurden neue 3,5-disubstituierte 4-Pyridone der allgemeinen Formel (I),

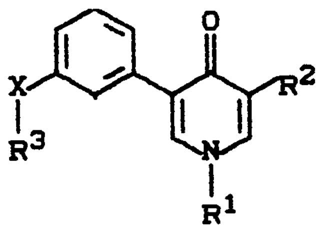

(I)

in welcher

$R^1$ für Hydroxy, Alkyl, Halogenalkyl, Alkenyl, Alkinyl, Alkoxy, Alkoxyalkyl oder Dialkylamino steht,

$R^2$ für gegebenenfalls substituiertes Triazolyl, Imidazolyl, Pyrazolyl oder Phenyl steht,

$R^3$ für Halogenalkyl oder Halogenphenyl steht, und

X für Sauerstoff oder Schwefel steht,

gefunden.

Weiterhin wurde gefunden, daß man die 3,5-disubstituierten 4-Pyridone der Formel (I) erhält, wenn man Ketone der Formel (II)

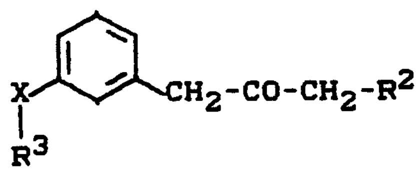

(II)

in welcher

$R^2$, $R^3$ und X die oben angegebenen Bedeutungen haben,

zunächst mit einem Formylierungsmittel in Gegenwart einer Base und in Gegenwart eines Verdünnungsmittels umsetzt, danach mit wässrigen Lösungen der Salze von Aminen der Formel (III),

$R^1$-$NH_2$ (III)

in welcher $R^1$ die oben angegebene Bedeutung hat,

in Gegenwart desselben Verdünnungsmittels in üblicher Weise versetzt und anschließend das Gemisch der beiden entstehenden Enamine der Formeln (IVa) und (IVb)

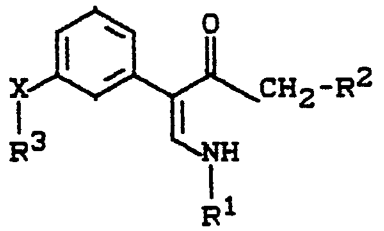

und

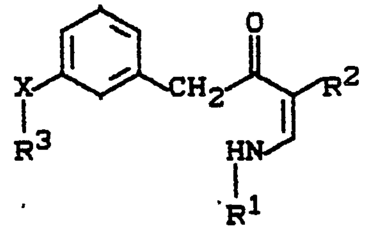

(IVa) (IVb

in welchen

$R^1$, $R^2$, $R^3$ und X die oben angegebenen Bedeutungen haben,

ohne Isolierung der gleichen Reaktionsfolge-Umsetzung mit dem gleichen Formylierungsmittel und dem gleichen Aminsalz unter den gleichen Reaktionsbedingungen unterwirft.

Außerdem wurde gefunden, daß die neuen 3,5-disubstituierten 4-Pyridone der Formel (I) gute herbizide, insbesondere selektiv-herbizide und pflanzenwachstumsregulatorische Eigenschaften aufweisen.

Überraschenderweise zeigen die erfindungsgemäßen 3,5-disubstituierten 4-Pyridone der allgemeinen Formel (I) neben einer deutlich höheren herbiziden Wirksamkeit gegenüber Unkräutern auch eine erheblich verbesserte Selektivität gegenüber wichtigen Kulturpflanzen, wie beispielsweise Baumwolle, als die aus dem Stand der Technik bekannten 3,5-disubstituierten 4-Pyridone, wie beispielsweise das 1-Methyl-3-phenyl-5-(3-trifluormethylphenyl)-4-pyridon, welche chemisch und wirkungsmäßig naheliegende Verbindungen sind.

Außerdem zeigen die erfindungsgemäßen Verbindungen der Formel (I) überraschenderweise eine starke pflanzenwuchsregulierende Wirkung.

Die erfindungsgemäßen 3,5-disubstituierten 4-Pyridone sind durch die Formel (I) allgemein definiert. Bevorzugt sind Verbindungen der Formel (I), in denen

$R^1$ für Hydroxy, Alkyl mit 1 bis 4 Kohlenstoffatomen, Halogenalkyl mit 1 bis 4 Kohlenstoffatomen und 1 bis 9 gleichen oder verschiedenen Halogenatomen, für Alkenyl oder Alkinyl mit jeweils 3 oder 4 Kohlenstoffatomen, für Alkoxy, Alkoxyalkyl sowie Dialkylamino mit jeweils 1 bis 4 Kohlenstoffatomen in den einzelnen Alkylteilen steht,

$R^2$ für gegebenenfalls einfach oder mehrfach, gleich oder verschieden substituiertes Triazolyl, Imidazolyl oder Pyrazolyl steht, wobei als Substituenten jeweils Halogen, Alkyl oder Alkoxy mit jeweils 1 bis 4 Kohlenstoffatomen in Frage kommen,

$R^3$ für einfach bis achtfach, gleich oder verschieden durch Chlor oder Fluor substituiertes Alkyl mit 1 bis 8 Kohlenstoffatomen oder für einfach bis fünffach durch Fluor substituiertes Phenyl steht und

X für Sauerstoff oder Schwefel steht.

Bevorzugt sind außerdem Verbindungen der Formel (I), in denen

$R^1$ für Hydroxy, Alkyl mit 1 bis 4 Kohlenstoffatomen, Halogenalkyl mit 1 bis 4 Kohlenstoffatomen und 1 bis 9 gleichen oder verschiedenen Fluor-und Chloratomen, für Alkenyl oder Alkinyl mit jeweils 3 oder 4 Kohlenstoffatomen, für Alkoxy, Alkoxyalkyl sowie Dialkylamino mit jeweils 1 bis 4 Kohlenstoffatomen in den einzelnen Alkylteilen steht,

$R^2$ für gegebenenfalls einfach oder mehrfach, gleich oder verschieden substituiertes Phenyl steht, wobei als Substituenten ausgewählt sind:

Halogen, Alkyl, Alkoxy und Alkylthio mit jeweils 1 bis 4 Kohlenstoffatomen sowie Halogenalkyl mit 1 oder 2 Kohlenstoffatomen und 1 bis 5 gleichen oder verschiedenen Halogenatomen, wie vorzugsweise Fluor und Chlor,

$R^3$ für einfach bis achtfach, gleich oder verschieden durch Chlor oder Fluor substituiertes Alkyl mit 1 bis 8 Kohlenstoffatomen oder für einfach bis fünffach durch Fluor substituiertes Phenyl steht und

X für Sauerstoff oder Schwefel steht

Besonders bevorzugt sind diejenigen Verbindungen der Formel (I), in denen

$R^1$ für Hydroxy, Methyl, Ethyl, i-Propyl, 1,1,1,-Trifluor-prop-2-yl, 2,2,2-Trifluor-eth-1-yl, Trifluormethyl, Allyl, Propargyl, Methoxy, Ethoxy, Methoxymethyl, Ethoxymethyl, Ethoxyethyl oder Dimethylamino steht,

$R^2$ für gegebenenfalls einfach oder zweifach, gleich oder verschieden substituiertes 1,2,4-Triazolyl, Imidazolyl oder Pyrazolyl steht, wobei als Substituenten jeweils Methyl, Methoxy, Fluor und Chlor in Frage kommen;

$R^3$ für einfach bis sechsfach durch Fluor substituiertes Alkyl mit 1 bis 6 Kohlenstoffatomen oder für einfach bis fünffach durch Fluor substituiertes Phenyl steht und

X für Sauerstoff oder Schwefel steht.

Besonders bevorzugt sind außerdem diejenigen Verbindungen der Formel (I), in denen

$R^1$ für Hydroxy, Methyl, Ethyl, i-Propyl, Trifluormethyl, 1,1,1-Trifluor-prop-2-yl, 2,2,2-Trifluor-eth -1-yl, Allyl, Propargyl, Methoxy, Ethoxy, Methoxymethyl, Ethoxymethyl, Ethoxyethyl oder Dimethylamino steht,

$R^2$ für gegebenenfalls einfach oder zweifach, gleich oder verschieden substituiertes Phenyl steht, wobei als Substituenten ausgewählt sind:

Fluor, Chlor, Brom, Methyl, Ethyl, i-Propyl, Methoxy, Ethoxy, i-Propoxy, Methylthio, Ethylthio, i-Propylthio und Trifluormethyl.

$R^3$ für einfach bis sechsfach durch Fluor substituiertes Alkyl mit 1 bis 6 Kohlenstoffatomen oder für einfach bis fünffach durch Fluor substituiertes Phenyl steht und

X für Sauerstoff und Schwefel steht.

Verwendet man beispielsweise 1-(3-Trifluormethoxyphenyl)-3-(4-chlorpyrazol-1-yl)-propan-2-on als Ausgangsstoff, Ameisensäureethylester als Formylierungsmittel und Methylamin-hydrochlorid als Aminkomponente, so kann der Reaktionsablauf des erfindungsgemäßen Verfahrens durch das folgende Formelschema wiedergegeben werden:

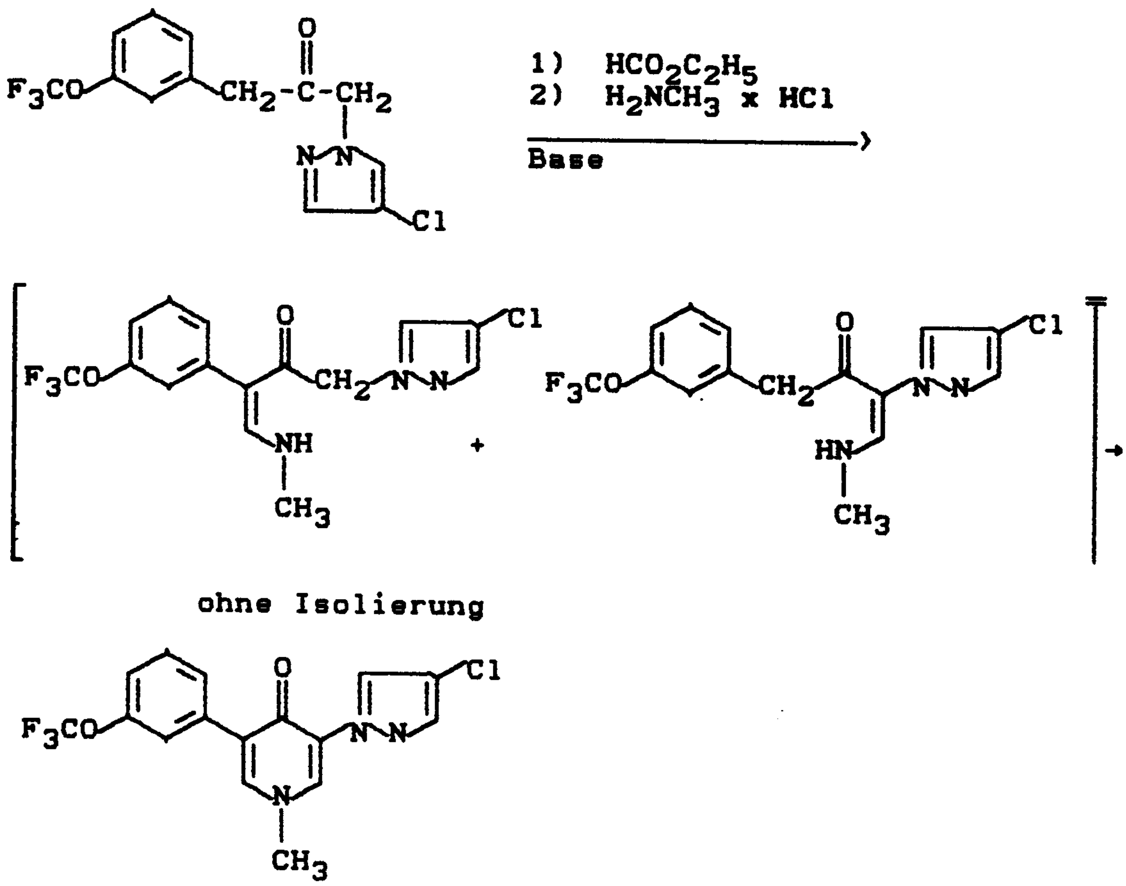


Die für die erfindungsgemäße Reaktion als Ausgangsstoffe zu verwendenden Ketone sind durch die Formel (II) allgemein definiert. In dieser Formel stehen $R^2$, $R^3$ und X vorzugsweise für die Reste, die bereits im Zusammenhang mit der Beschreibung der erfindungsgemäßen Stoffe der Formel (I) vorzugsweise für diese Substituenten genannt wurden.

Die Ketone der Formel (II) sind bekannt bzw. können in bekannter Art und Weise erhalten werden, indem man z.B. Benzylcyanide der Formel -(V)

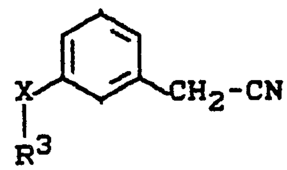

(V)

in welcher

$R^3$ und X die oben angegebenen Bedeutungen haben,

mit Essigsäureester-Derivaten der Formel (VI)

$RO-CO-CH_2-R^2$ (VI)

in welcher

R für Alkyl mit 1 bis 4 Kohlenstoffatomen steht und

$R^2$ die oben angegebene Bedeutung hat,

in Gegenwart einer Base, wie z.B. Natriummethylat, und in Gegenwart eines inerten organischen Lösungsmittels, wie z.B. Ethanol, vorzugsweise bei der Siedetemperatur des verwendeten Lösungsmittels umsetzt und anschließend im System Schwefelsäure/Wasser hydrolisiert (vergleiche auch die Herstellungsbeispiele).

Die Benzylcyanide der Formel (V) und die Essigsäureester-Derivate der Formel (VI) sind allgemein bekannte Verbindungen der organischen Chemie und können nach bekannten Verfahren in analoger Weise hergestellt werden.

Als Formylierungsmittel können für die erfindungsgemäße Reaktion alle üblicherweise verwendbaren Formylierungsreagenzien eingesetzt werden. Hierzu gehören vorzugsweise Ameisensäureester, wie z.B. Ameisensäuremethyl-und -ethylester.

Die weiterhin für die erfindungsgemäße Reaktion als Ausgangsstoffe benötigten Amine sind durch die Formel (III) allgemein definiert. In dieser Formel steht $R^1$ vorzugsweise für die Reste, die bereits im Zusammenhang mit der Beschreibung der erfindungsgemäßen Stoffe der Formel (I) vorzugsweise für diesen Substituenten genannt wurden. Die Amine der Formel (III) werden bevorzugt in Form ihrer Salze eingesetzt, wie z.B. als Hydrohalogenide oder Hydrosulfate.

Die Amine der Formel (III) sind allgemein bekannte Verbindungen der organischen Chemie.

Die erfindungsgemäße Reaktion wird in Gegenwart einer Base durchgeführt. Hierbei können alle üblicherweise verwendbaren organischen und anorganischen Basen eingesetzt werden, vorzugsweise verwendet man Alkoholate, wie z.B. Natriummethylat oder -ethylat oder Kaliumtert.-butylat, oder Hydride, wie Natrium-oder Kaliumhydrid.

Als Verdünnungsmittel kommen für die erfindungsgemäße Umsetzung vorzugsweise mit Wasser mischbare organische Lösungsmittel infrage. Hierzu gehören vorzugsweise Ether, wie insbesondere Tetrahydrofuran oder Dioxan.

Die Reaktionstemperaturen können bei der Durchführung des erfindungsgemäßen Verfahrens in einem bestimmten Bereich variiert werden. Im allgemeinen arbeitet man bei Temperaturen zwischen -20 und +60°C, vorzugsweise bei -10 bis +40°C.

Bei der Durchführung des erfindungsgemäßen Verfahrens setzt man zunächst auf 1 Mol Keton der Formel (II) einen Überschuß an Formylierungsmittel, insbesondere die zwei-bis dreifache Menge, einen Überschuß an Base, insbesondere die zwei-bis dreifache Menge, und einen Überschuß an Amin der Formel (III), insbesondere ebenfalls die zwei-bis dreifache Menge, ein. Die gleichen überschüssigen Mengen an Formylierungsmittel, Base und Amin werden bei der Wiederholung de erfindungsgemäßen Reaktionsfolge (Ringschlußreaktion) eingesetzt. Die Aufarbeitung und Isolierung der Endprodukte der Formel (I) erfolgt in allgemein üblicher Art und Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea,

Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Datyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonderes gutem Erfolg zur selektiven Bekämpfung mono-und dikotyler Unkräuter in mono-und dikotylen Nutzpflanzenkulturen wie insbesondere Baumwollkulturen einsetzen.

Die erfindungsgemäßen Wirkstoffe greifen in den Metabolismus der Pflanzen ein und können deshalb als Wachstumsregulatoren eingesetzt werden. So können z. B. die erfindungsgemäßen Wirkstoffe zur Entlaubung und zum Austrocknen der Blätter der Baumwolle eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park-und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen-oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekänpfung Verwendung finden, wobei Fertigformulierung oder Tankmischung möglich ist.

Für die Mischung können bekannte Herbizide verwendet werden wie z.B. Harnstoffe, wie N-(3-Trifluormethylphenyl)-N',N'-dimethylharnstoff, Nitroaniline, wie N,N-Di-(n-propyl)-2,6-dinitro-4-trifluormethylanilin; Chloracetanilide, wie 2',6'-Diethyl-N-(methoxymethyl)-2-chloracetanilid und 2-Ethyl-6-methyl-N-(1'-methyl-2'-methoxyethyl)-chloracetanilid; Thiolcarbamate, wie 2,3,3-Trichlorallyldiisopropylthiocarbamat; Benzylether, wie exo-1-Methyl-4-(1-methylethyl)-2-(2-methylphenylmethoxy)-7-oxabicyclo[2,2,1]-hepatan; Oxyessigsäureamide, wie 2-(2-Benzothiazolyloxy)-N-methyl-N-phenylacetamid; sowie Aryl-oder Heteroaryloxy-phenoxypropionsäuren, wie 2-{4-[(3-Chlor-5-trifluormethyl-2-pyridinyl)-oxy]-phenoxy}-propansäure. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen oder Stäuben.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann beim Einsatz der erfindungsgemäßen Verbindungen als Herbizide in größeren Bereich schwanken. Sie hängt im wesentlichen von der Art des gewünschten Effektes ab. Im allgemeinen liegen die Aufwandmengen zwischen 0,01 und 10 kg Wirkstoff pro ha, vorzugsweise zwischen 0,05 und 5 kg/ha.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Herstellung und Verwendung der erfindungsgemäßen Wirkstoffe gehen aus den folgenden Beispielen hervor.

Herstellungsbeispiele

Beispiel 1

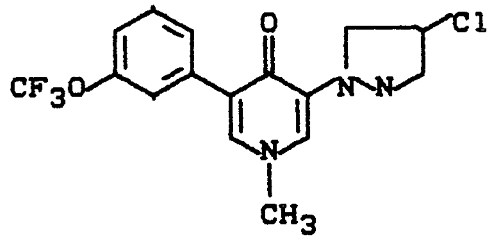

37,37 g (0,68 Mol) Natriummethylat in 500 ml Tetrahydrofuran werden mit 109 g (0,34 Mol) 1-(3-Trifluormethoxyphenyl)-3-(4-chlorpyrazol-1-yl)-propan-2-on versetzt. Zu diesem Reaktionsgemisch werden bei 10°C bis 15°C 70,81 ml (0,854 Mol) Ameisensäureethylester innerhalb von 30 Minuten zugegeben. Nach einer Stunde Rühren werden weitere 56,94 ml (0,683 Mol) Ameisensäureethylester zugetropft. Das Reaktionsgemisch wird über Nacht bei Raumtemperatur gerührt. Danach wird eine Lösung von 56,9 g Methylamin-hydrochlorid in 150 ml Wasser zugegeben und bei 30°C eine halbe Stunde gerührt.

Das Zweiphasengemisch wird mit Methylenchlorid versetzt, die organische Phase abgetrennt, getrocknet und eingeengt. Der Rückstand wird erneut in Methylenchlorid aufgenommen, getrocknet und eingeengt. Der dann erhaltene Rückstand wird mit Ether versetzt und der kristalline Niederschlag abgesaugt.

Man erhält 43 g (34 % der Theorie) an 1-Methyl-3-(4-chlorpyrazol-1-yl)-5-(3-trifluormethoxyphenyl)-pyrid-4-on vom Schmelzpunkt 109°C.

Herstellung der Ausgangsprodukte

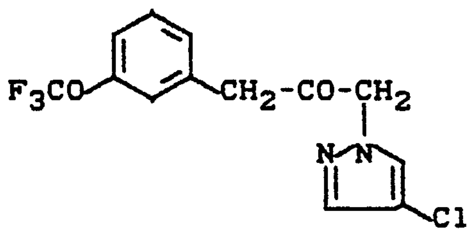

160 g (0,42 Mol) 1-Cyano-1-(3-trifluormethylphenyl)-3-(4-chlorpyrazol-1-yl)-propan-2-on-hydrochlorid werden mit 100 ml konzentrierter Schwefelsäure versetzt, wobei die Temperatur auf 80°C ansteigt. Anschließend wird bei 100-110°C bis zur Beendigung der $CO_2$-Entwicklung (ca. 45 Minuten) gerührt.

Man läßt das Reaktionsgemisch abkühlen, verdünnt mit 50 ml Wasser, stellt mit 40-prozentiger Natronlauge auf einen pH-Wert von 7-8 ein, extrahiert mit Ether, wäscht die Etherphase mit einer Natriumchlorid-Lösung, trocknet und engt ein.

Man erhält 117 g (87 % der Theorie) an 1-(3-Trifluormethoxyphenyl)-3-(4-chlorpyrazol-1-yl)-propan-2-on vom Schmelzpunkt 60-61°C.

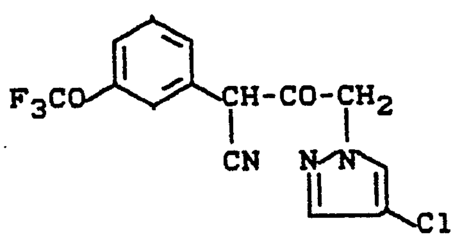

Zu einer frisch hergestellten Lösung von 24,6 g (1,07 Mol) Natrium in 500 ml Ethanol wird bei 70°C unter Rühren innerhalb von 6 Stunden ein Gemisch von 100 g (0,53 Mol) 3-Trifluormethoxybenzylcyanid und 120 g (0,64 Mol) 4-Chlorpyrazol-1-yl-essigsäureethylester getropft und anschließend 12 Stunden unter Rückfluß erhitzt und gerührt. Der nach dem Abkühlen ausgefallene Niederschlag wird in Wasser gelöst und zur Entfernung nicht umgesetzter Edukte mit Ether extrahiert. Die

wässrige Phase wird mit verdünnter Salzsäure bis pH 3 angesäuert, der Niederschlag abgesaugt, das Filtrat mit Ether extrahiert, der Extrakt getrocknet und eingeengt. Die Niederschläge werden vereinigt.

Man erhält 169 g (87 % der Theorie) an 1-Cyano-1-(3-trifluormethoxyphenyl)-3-(4-chlorpyrazol-1-yl)-propan-2-on vom Schmelzpunkt 108°C.

In entsprechender Weise werden auch die folgenden 3,5-disubstituierten 4-Pyridone der allgemeinen Formel (I)

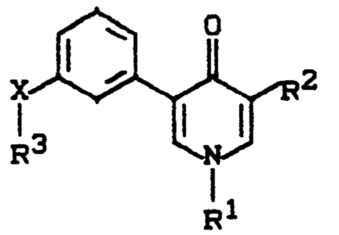

(I)

erhalten:

Tabelle 1:

| Bsp. Nr. | $R^1$ | $R^2$ | $R^3$ | X | physikalische Daten |
|---|---|---|---|---|---|
| 2 | $-CH_3$ | 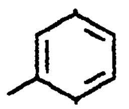 | $-CF_3$ | O | $n_D^{30}$ = 1,5530 |
| 3 | $-CH_3$ | 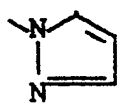 | $-CF_3$ | O | $n_D^{30}$ = 1,5589 |
| 4 | $-CH_3$ | 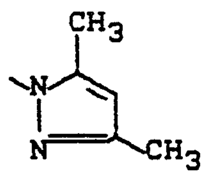 | $-CF_3$ | O | Fp = 162° C |
| 5 | $-CH_3$ | 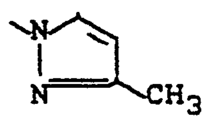 | $-CF_3$ | O | Fp = 114-115° C |
| 6 | $-CH_3$ | 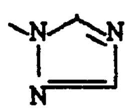 | $-CF_3$ | O | Fp = 143° C |
| 7 | $-CH_3$ | 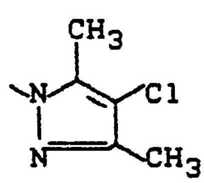 | $-CF_3$ | O | Fp = 204° C |

Tabelle 1:

| Bsp. Nr. | $R^1$ | $R^2$ | $R^3$ | X | physikalische Daten |
|---|---|---|---|---|---|
| 8 | $-CH_3$ | 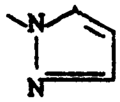 | $-CF_3$ | S | Öl |
| 9 | $-CH_2-CF_3$ | 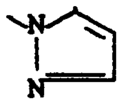 | $-CF_3$ | O | Öl |
| 10 | 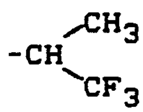 | 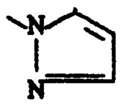 | $-CF_3$ | O | Öl |
| 11 | OH | 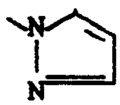 | $-CF_3$ | O | Fp. = 146-147° C |

Anwendungsbeispiele:

In den folgenden Anwendungsbeispielen wurde die nachstehend aufgeführte Verbindung als Vergleichssubstanz eingesetzt:

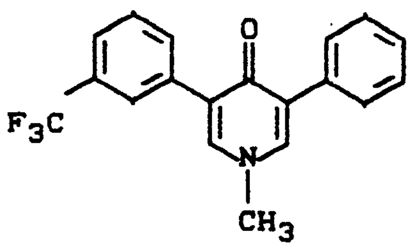

(A)

1-Methyl-3-phenyl-5-(3-trifluormethylphenyl)-4-pyridon (bekannt aus DE-OS 25 37 753).

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in %

Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigt z.B. die Verbindung gemäß dem Herstellungsbeispiel 1, 2 und 3 neben einer deutlich besseren Wirksamkeit gegen monokotyle Unkräuter eine wesentliche besse re Verträglichkeit in Nutzpflanzen, wieinsbesondere Baumwolle, als die Vergleichssubstanz (A).

Beispiel B

Entlaubung und Austrocknung der Blätter bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall

\+ leichtes Austrocknen der Blätter, geringer Blattfall

\+ + starkes Austrocknen der Blätter, starker Blattfall

\+ + + sehr starkes Austrocknen der Blätter, sehr starker Blattfall

Eine sehr gute Wirksamkeit im Vergleich zur unbehandelten Kontrolle zeigt in diesem Test z.B. die Verbindung gemäß Herstellungsbeispiel 1.

**Ansprüche**

1. 3,5-Disubstituierte 4-Pyridone der Formel (I),

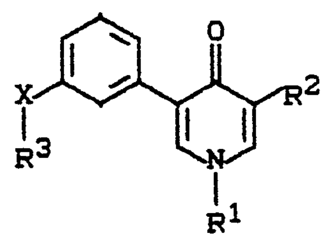

(I)

in welcher

$R^1$ für Hydroxy, Alkyl, Halogenalkyl, Alkenyl, Alkinyl, Alkoxy, Alkoxyalkyl oder Dialkylamino steht,

$R^2$ für gegebenenfalls substituiertes Triazolyl, Imidazolyl, Pyrazolyl oder Phenyl steht,

$R^3$ für Halogenalkyl oder Halogenphenyl steht, und

X für Sauerstoff oder Schwefel steht.

2. 3,5-Disubstituierte 4-Pyridone der Formel (I) gemäß Anspruch 1, in welcher

$R^1$ für Hydroxy, Alkyl mit 1 bis 4 Kohlenstoffatomen, Halogenalkyl mit 1 bis 4 Kohlenstoffatomen und 1 bis 9 gleichen oder verschiedenen Fluor-und Chloratomen, für Alkenyl oder Alkinyl mit jeweils 3 oder 4 Kohlenstoffatomen, für Alkoxy, Alkoxyalkyl sowie Dialkylamino mit jeweils 1 bis 4 Kohlenstoffatomen in den einzelnen Alkylteilen steht,

$R^2$ für gegebenenfalls einfach oder mehrfach, gleich oder verschieden substituiertes Triazolyl, Imidazolyl oder Pyrazolyl steht, wobei als Substituenten jeweils Halogen, Alkyl oder Alkoxy mit jeweils 1 bis 4 Kohlenstoffatomen in Frage kommen,

$R^3$ für einfach bis achtfach, gleich oder verschieden durch Chlor oder Fluor substituiertes Alkyl mit 1 bis

8 Kohlenstoffatomen oder für einfach bis fünffach durch Fluor substituiertes Phenyl steht und

X für Sauerstoff oder Schwefel steht.

3. 3,5-Disubstituierte 4-Pyridone der Formel (I) gemäß Anspruch 1,

in welcher

$R^1$ für Hydroxy, Alkyl mit 1 bis 4 Kohlenstoffatomen, Halogenalkyl mit 1 bis 4 Kohlenstoffatomen und 1 bis 9 gleichen oder verschiedenen Fluor-und Chloratomen, für Alkenyl oder Alkinyl mit jeweils 3 oder 4 Kohlenstoffatomen, für Alkoxy, Alkoxyalkyl sowie Dialkylamino mit jeweils 1 bis 4 Kohlenstoffatomen in den einzelnen Alkylteilen steht,

$R^2$ für gegebenenfalls einfach oder mehrfach, gleich oder verschieden substitutiertes Phenyl steht, wobei als Substituenten ausgewählt sind:

Halogen, Alkyl, Alkoxy und Alkylthio mit jeweils 1 bis 4 Kohlenstoffatomen sowie Halogenalkyl mit 1 bis 2 Kohlenstoffatomen und 1 oder 5 gleichen oder verschiedenen Halogenatomen,

$R^3$ für einfach bis achtfach, gleich oder verschieden durch Chlor oder Fluor substituiertes Alkyl mit 1 bis 8 Kohlenstoffatomen oder für einfach bis fünffach durch Fluor substituiertes Phenyl steht und

X für Sauerstoff oder Schwefel steht.

4. Verfahren zur Herstellung von 3,5-disubstituierten 4-Pyridone der Formel (I),

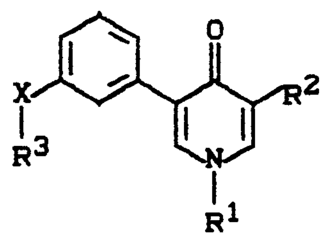

(I)

in welcher

$R^1$ für Hydroxy, Alkyl, Halogenalkyl, Alkenyl, Alkinyl, Alkoxy, Alkoxyalkyl oder Dialkylamino steht,

$R^2$ für gegebenenfalls substituiertes Triazolyl, Imidazolyl, Pyrazolyl oder Phenyl steht,

$R^3$ für Halogenalkyl oder Halogenphenyl steht, und

X für Sauerstoff oder Schwefel steht,

dadurch gekennzeichnet, daß man Ketone der Formel (II),

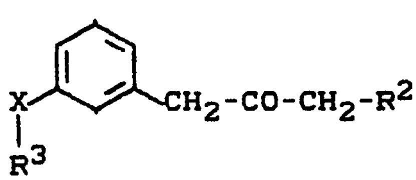

(II)

in welcher

$R^2$, $R^3$ und X die oben angegebenen Bedeutungen haben,

zunächst mit einem Formylierungsmittel in Gegenwart einer Base und in Gegenwart eines Verdünnungsmittels umsetzt, danach mit wässrigen Lösungen der Salze von Aminen der Formel (III),

$R^1$ -$NH_2$ (III)

in welcher $R^1$ die oben angegebene Bedeutung hat,

in Gegenwart desselben Verdünnungsmittels versetzt und anschließend das Gemisch der beiden entstehenden Enamine der Formeln (IVa) und (IVb)

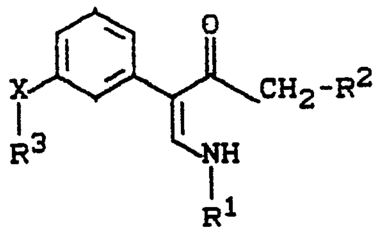

und

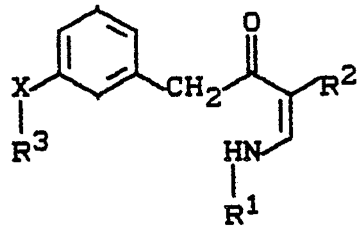

(IVa) (IVb)

in welchen

$R^1$, $R^2$, $R^3$ und X die oben angegebenen Bedeutungen haben,

ohne Isolierung der gleichen Reaktionsfolge-Umsetzung mit dem gleichen Formylierungsmittel und dem gleichen Aminsalz unter den gleichen Reaktionsbedingungen unterwirft.

5. Herbizide und pflanzenwuchsregulierende Mittel, gekennzeichnet durch einen Gehalt an mindestens eine 3,5-disubstituierten 4-Pyridon der Formel (I) gemäß den Ansprüchen 1 bis 4.

6. Verfahren zur Bekämpfung von Unkräutern, dadurch gekennzeichnet, daß man 3,5-disubstituierte 4-Pyridone der Formel (I) gemäß den Ansprüchen 1 bis 4 auf Unkräuter und/oder ihren Lebensraum einwirken läßt.

7. Verwendung von 3,5-disubstituierten 4-Pyridonen der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man 3,5-disubstituierte 4-Pyridone der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 86 11 1730

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 283 130 (ELI LILLY & CO.) * Beispiele 112,139-144; Ansprüche 1,3,4 (Seite 133, Zeilen 4,5) 5-8 * | 1,3-8 | C 07 D 213/68<br>C 07 D 213/89<br>C 07 D 401/04<br>A 01 N 43/40<br>A 01 N 43/48<br>A 01 N 43/50<br>A 01 N 43/647//<br>C 07 D 231/16 |
| Y | * Ansprüche 1, 3-8; & DE - A - 25 37753 (Kat. D) * | 1-8 | |
| | --- | | |
| Y | EP-A-0 073 999 (BAYER AG) * insgesamt * | 1-8 | |
| | --- | | |
| X | FR-A-2 395 996 (ELI LILLY & CO.) * Beispiel 77; Seite 32, Zeile 21 - Seite 33, Zeile 21, Tabelle 2, Seite 41, Verbindung 77; Ansprüche 1,2,4,9 (Seite 66, Zeilen 13,14), 14 * | 1,3-8 | **RECHERCHIERTE SACHGEBIETE (Int. Cl.4)** |
| | --- | | C 07 D 213/00<br>C 07 D 401/00<br>C 07 D 521/00<br>A 01 N 43/00 |
| X | US-A-4 235 619 (H.M. TAYLOR) * Spalte 6, Zeilen 55,56; Spalte 7, Zeilen 15,16-55-58; Spalte 9, Zeilen 57,58; Beispiele 131,133,149; Spalte 39, Zeile 62 - Spalte 43, Zeile 4 * | 1,3-8 | |
| | ----- | | |

Der vorliegende Recherchenbericht wurde für alle Patentansprüche erstellt.

| Recherchenort | Abschlußdatum der Recherche | Prüfer |
|---|---|---|
| BERLIN | 06-11-1986 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82